# FASCICULE DE BREVET EUROPEEN

(11) **EP 3 151 816 B1**
(45) Date de publication et mention de la délivrance du brevet: **24.07.2019**
(21) Numéro de dépôt: 15732844.4
(22) Date de dépôt: 04.06.2015
(51) Int. Cl.: A61K 31/075, A61K 31/201, A61K 31/455, A61K 33/30, A61P 17/00, A61P 17/10, A61K 8/00, A61K 8/27, A61K 8/36, A61K 8/49, A61Q 19/00, A61Q 19/02, A61K 8/19

(54) **COMPOSITION DERMATOLOGIQUE À BASE DE NIACINAMIDE**
NIACINAMIDBASIERTE DERMATOLOGISCHE ZUSAMMENSETZUNG
NIACINAMIDE-BASED DERMATOLOGICAL COMPOSITION

(30) Priorité: 06.06.2014 FR 1455175
(43) Date de publication de la demande: 12.04.2017
(73) Titulaire: Espa SA-Experts in Science&Product Achievements, 31100 Toulouse (FR)
(72) Inventeur: HUET, Pierre, F-31100 Toulouse (FR)
(74) Mandataire: Cabinet Plasseraud
(86) Numéro de dépôt international: PCT/FR2015/051478
(87) Numéro de publication internationale: WO 2015/185867

(56) Documents cités:
- WO-A1-2012/149600
- CA-A1- 2 701 378
- CN-A- 101 468 006
- DE-A1-102008 012 988
- US-A1- 2010 249 244

## Description

La présente invention concerne des compositions topiques à base de niacinamide et d'acide oléique ainsi que leur utilisation d'une part pour le traitement thérapeutique des troubles cutanés et d'autre part pour le traitement cosmétique des imperfections cutanées, et/ou l'éclaircissement de la peau.

L'acné est une des maladies les plus fréquentes de l'adolescence, 80 à 90 % des jeunes (filles et garçons) présentent une forme d'acné. En raison d'une stimulation androgénique physiologiquement plus importante et d'une séborrhée plus marquée, le jeune homme aura tendance à développer une acné plus fréquemment que la jeune fille. Environ 90 % des acnés régressent avant la trentaine.

Quatre facteurs sont actuellement considérés comme responsables de l'apparition de l'acné :
- La surproduction de sébum, fortement dépendante de la sécrétion hormonale.
- L'hyperplasie des glandes sébacées.
- L'hyperkératose du follicule de la glande sébacée conduisant à l'obstruction du canal folliculaire par la kératine.
- La colonisation microbienne du follicule par la bactérie *Propionibacterium acnes,* et les réactions immunologiques et inflammatoires qu'elle provoque.

Tous les experts admettent actuellement que le microcomédon est la lésion initiale de l'acné. Il se développe en raison d'une production sébacée excessive, hormono-dépendante, et de troubles de la kératinisation dans le follicule. Les glandes sébacées de patients acnéiques montrent une augmentation de récepteurs aux androgènes et ces récepteurs ont une sensibilité supérieure à l'effet des androgènes (Toyoda M et coll, Pathogenesis of acne. Med Electron Microsc 2001 ; 34 p 29-40).

Ensuite se développent des comédons d'aspect foncé, ouverts, et des comédons clairs, fermés. Leur colonisation massive par *Propionibacterium acnes* induit une transformation en papules inflammatoires. Le développement de cette réaction inflammatoire implique les lymphocytes T CD4.

La rupture de la paroi du follicule sous l'effet de l'inflammation massive, aboutit à l'attraction de granulocytes neutrophiles et à la libération de médiateurs pro-inflammatoires.

Les principaux actifs utilisés dans le traitement de l'acné juvénile par voie topique sont les suivants :
- Le peroxyde de benzoyle : est l'un des traitements locaux les plus anciens et les plus éprouvés de l'acné. Il a été démontré que le peroxyde de benzoyle, inducteur de radicaux libres, peut potentialiser le développement du cancer cutané chez la souris. De plus cet actif est autorisé en cosmétique uniquement pour la pose des ongles artificiels.
- Les rétinoïdes topiques :
   Par exemples : la Trétinoine (Retin A), l'adapalen (Differin) ces deux produits sont commercialisés comme médicaments et ne peuvent donc être utilisés dans un produit cosmétique.
   L'un des principaux inconvénients des rétinoïdes topiques, réside en la survenue rapide de récidives à l'arrêt du traitement.
- L'acide azélaïque :
   De part ses mécanismes d'action théoriques, l'acide azélaïque se présentait comme un actif très prometteur. Il exerce simultanément des effets comédolytiques, bactériostatiques et anti-inflammatoires, mais malheureusement son utilisation en mono thérapie s'est révélée décevante, même à des doses élevées.
- Les antibiotiques topiques :
   Les représentants classiques de cette famille sont la clindamycine et l'érythromycine. Ces deux principes actifs sont commercialisés comme médicaments et ne peuvent être utilisés dans un produit cosmétique.

Il est également décrit des compositions permettant de traiter des troubles cutanées tels que l'acné ou les troubles de la pigmentation comprenant du dihydroeugenol et/ou de l'isoeugénol et/ou l'éthylvanilline, ainsi que du niacinamide et du dimethyl isosorbide (US2010/249244, DE102008012988, CA2701378, WO2012/149600, CN101468006).

La présente invention concerne une composition dermocosmétique utilisable en cosmétologie utile dans le traitement des peaux à tendance acnéique et dans le traitement de l'acné juvénile, ainsi que dans le traitement des troubles de la pigmentation.

### Résumé de l'invention :

Un premier objet de l'invention consiste en une composition comprenant : de la niacinamide, de l'acide oléique et du diméthylisosorbide.

La niacinamide (nicotinamide, vitamine PP ou vitamine B3) est un co-facteur enzymatique. Outre son intérêt nutritionnel, ce produit possède des propriétés : kératolytiques, anti-séborrhéiques et anti-inflammatoires, qui en font un actif de choix dans le domaine cosmétique.

L'inventeur a montré qu'en associant l'acide oléique et le diméthylisosorbide à la niacinamide, ces composés agissaient en synergie et les propriétés anti-inflammatoires de la niacinamide et de l'acide oléique étaient potentialisées. Le diméthylisosorbide est utilisé, quant à lui, comme vecteur de pénétration.

Outre son action anti-inflammatoire, la composition selon l'invention possède également des propriétés dépigmentantes.

L'inventeur a montré que du fait de ses propriétés anti-inflammatoires et dépigmentantes, la composition selon l'invention était particulièrement efficace pour le traitement des troubles cutanés notamment pour le traitement de l'acné et des troubles de la pigmentation tels que l'hyperpigmentation.

Alternativement, la composition selon l'invention peut être utilisée à des fins cosmétiques. Ainsi, la composition peut être utilisée afin d'éclaircir la peau, d'homogénéiser le teint, de supprimer les imperfections cutanées telles que les éphélides, taches brunes.

### Description détaillée :

La présente invention concerne une composition topique comprenant : de la niacinamide, de l'acide oléique et du diméthylisosorbide.

Typiquement, cette composition est une composition topique en vue d'une application cutanée.

La composition peut comprendre en outre de la calamine. La calamine est utilisée comme protecteur cutané.

De préférence, la calamine est ajoutée à la composition lorsque cette dernière est utilisée pour le traitement thérapeutique de l'acné. La calamine peut également être ajoutée à la composition lorsque cette dernière est utilisée pour le traitement cosmétique des peaux à tendance acnéique.

La composition peut en outre comprendre un ou plusieurs agents dépigmentant, de préférence non irritants.

Des exemples d'agents dépigmentant sont l'acide octadécenedioïque et/ou l'undecylenoyl phenylalanine. Les agents dépigmentant peuvent être ajoutés pour renforcer l'action dépigmentante de la composition selon l'invention. La composition peut également comprendre des agents anti-acnéiques additionnels tels que du peroxyde de benzoyle, un dérivé rétinoïque, de l'acide azélaïque et/un antibiotique.

Selon un mode de réalisation, la concentration en niacinamide de la composition est comprise entre 0,3 % et 3 % en poids de la composition, de préférence entre 0,35% et 2,8%, entre 0,5% et 2,5% ou entre 1% et 2%.

Selon un mode de réalisation préféré, la concentration en niacinamide est de 1% ou de 2% en poids de la composition

Selon un mode de réalisation, la concentration en acide oléique est comprise entre 0,3 % et 3 % en poids de la composition, de préférence entre 0,5% et 2,8%, entre 0,5% et 2,5% ou entre 1% et 2%.

Selon un mode de réalisation préféré, la concentration en l'acide oléique est de 0,5% ou de 2% en poids de la composition.

Selon un mode de réalisation, le ratio de la concentration en poids de la niacinamide sur celle de l'acide oléique est compris entre 0,5 et 2. De préférence, le pourcentage en poids de la niacinamide est égal à celui de l'acide oléique.

Selon un mode de réalisation, la concentration en diméthyl isosorbide est comprise entre 0,1 % et 2 % en poids de la composition, de préférence entre 0,14% et 2%, entre 0,5% et 2% ou entre 1% et 2%.

Selon un mode de réalisation plus préféré, la concentration en diméthyl isosorbide est de 2% en poids de la composition

Selon un mode de réalisation, la concentration en calamine est comprise entre 0,3% et 7 % en poids de la composition, de préférence entre 0,3% et 6,6%, entre 0,5% et 5%, entre 1,5% et 4,5% ou entre 2,5% et 3,5%.

Si la composition est utilisée pour le traitement des troubles de la pigmentation (éclaircissement du teint, taches brunes, lentigos solaires, éphélides, lucites) un ou plusieurs agents dépigmentant non irritants : l'acide octadécenedioïque et/ou l'undecylenoyl phenylalanine peuvent être ajoutés pour renforcer l'action dépigmentante.

Selon un mode de réalisation, la concentration en acide octadécenedioïque est comprise entre 0,5 % et 2 % en poids de la composition, de préférence entre 0,7% et 2% ou entre 1% et 2%.

Selon un mode réalisation, la concentration en undécylenoyl phenylalanine est comprise entre 1 % et 2 % en poids de la composition, de préférence de 1% ou 2%, plus préférentiellement de 2%.

La composition est typiquement une composition dermatologique. Elle peut comprendre, en outre, des excipients, en particulier des excipients dermatologiquement acceptables. Des excipients dermatologiquement acceptables sont bien connus dans l'état de la technique. Des exemples de tels excipients sont donnés aux exemples 1 à 10.

La composition peut être sous forme de solution, d'émulsion, de suspension de gel ou de dispersion.

Les formulations topiques préférées de la composition selon l'invention sont une lotion, une crème, un gel, une essence, un baume ou un stick.

La composition selon l'invention peut être utilisée à des fins thérapeutiques.

Ainsi, l'invention concerne également un procédé de traitement thérapeutique qui consiste à appliquer sur la peau d'un patient ladite composition.

En particulier, l'invention concerne de ladite composition pour son utilisation dans le traitement des dermatoses et autres des troubles cutanées.

Les troubles cutanés peuvent être de l'acné ou des troubles de la pigmentation tels que l'hyperpigmentation.

L'hyperpigmentation peut, notamment, être due à des lucites.

Selon un mode de réalisation préféré, ladite composition est pour une utilisation dans le traitement de l'acné.

L'invention concerne en particulier un procédé de traitement thérapeutique comprenant une étape d'application de la composition selon l'invention sur la peau d'un patient.

L'invention concerne aussi l'utilisation de la composition selon l'invention pour obtenir un produit de traitement topique destiné à traiter les troubles cutanés.

Alternativement, la composition selon l'invention peut être utilisée à des fins cosmétiques et non thérapeutique.

Ainsi, l'invention concerne un procédé de traitement cosmétique comprenant une étape d'application de la composition selon l'invention sur la peau d'un sujet.

L'invention concerne, notamment, un procédé de traitement cosmétique non thérapeutique qui consiste à appliquer sur la peau d'un sujet ladite composition pour éclaircir sa peau et/ou pour éliminer les imperfections cutanées.

En particulier, l'invention concerne l'utilisation d'une composition selon l'invention pour le traitement cosmétique des imperfections cutanées.

Les imperfections cutanées peuvent être par exemple des éphélides, des mélasmas, des lentigos solaires et/ou des taches brunes.

Les exemples qui suivent illustrent la présente demande.

### EXEMPLES

### EXEMPLES DE FORMULATIONS :

-A : Les compositions topiques utilisées dans le traitement des peaux à tendance acnéique et/ou l'acné selon la présente invention sont illustrées par les exemples 1 à 5 non limitatifs suivants :

**-Exemple 1 : Acne Cleanser**

| Principes actifs : | |
|---|---|
| Niacinamide : | 2,8 g |
| Acide oléique : | 2,8 g |
| Dimethyl isosorbide : | 0,70 g |
| Calamine : | 4,45 g |

| Autres excipients : | |
|---|---|
| Glycérine : | 0,5 à 1 g |
| Osmocide 3 Sederma: glycérine, eau, PEG 8, caprylyl glycol, sodium polyacrylate: | 6 à 8 g |
| Simulgel EG Seppic : sodium acrylate/sodium acryloylmethyltaurate, isohexadecane, polysorbate 80: | 2 à 3 g |
| Sepinov EMT 10: Seppic : hydroxyethylacrylate/ sodium acryloyldimethyltaurate copolymer: | 0,5 à 1,5g |
| DUB ININ B: Isononyl isononanoate: | 3 à 5 g |
| Huile d'abricot raffinée: | 9 à 13 g |
| Dehyton K COS : BASF: cocamidopropylbetaine: | 2 à 6 g |
| Proteol oat PF: Seppic: sodium lauroyl oat amino acids: | 2 à 5 g |
| Texapon NSO-IS: BASF: sodium laureth sulfate: | 4 à 6 g |
| Crothix liquid -LQ-RB: Croda: PEG-150 pentaerythrytyl tetrastearate et PEG-6 caprylic/capricglycerides: | 0,6 à 0,8g |
| Sharomix 721 : dehydroacetic acid/ benzylalcohol: | 0,8 à 1,1g |
| Eau déminéralisée QSP | 100 g |

**-Exemple 2 : Acné crème**

| Principes actifs : | |
|---|---|
| Niacinamide : | 2 g |
| Acide oléîque : | 2 g |
| Diméthyl isosorbide : | 1 g |
| Calamine : | 3 g |
| Acide octadécenedioïque : | 1 g |

| Autres excipients : | |
|---|---|
| Osmocide 3 :glycerine eau PEG 8 caprylylglycol sodium polyacrylate : | 4 à 6 g |
| DUB ININ B : isononyl isononanoate : | 3 à 5 g |
| DC9510:Dimethicone/VinylDimethicone Crosspolymer and C12-14 Pareth-12: | 1 à 2g |
| Aquaxyl : Seppic : xylitylglucoside, anhydroxylitol, xylitol : | 0,8 à 1,2 g |
| Huile d'abricot raffinée : | 10 à 14 g |
| Sharomix : dehydro acetic acid/ benzyl alcohol : | 0,6 à 1 g |
| Simulgel EG: Seppic : sodium acrylate/sodium acryloylmethyltaurate, isohexadecane, polysorbate 80: | 4 à 6 g |
| Sepinov EMT 10: Seppic :hydroxyethylacrylate/sodium acryloyldimethyltaurate copolymer : | 0,8 à 1,2 g |
| Eau déminéralisée QSP: | 100 g |

**-Exemple 3 : Acné sérum**

| Principes actifs : | |
|---|---|
| Niacinamide : | 2 g |
| Acide oléïque : | 2 g |
| Diméthyl isosorbide : | 1 g |
| Calamine : | 6,6 g |
| Acide octadécenedioïque : | 1 g |

| Autres excipients : | |
|---|---|
| Osmocide 3 :Sederma : glycérine, eau, PEG 8, caprylylglycol, sodium polyacrylate : | 4 à 6 g |
| DUB ININ B : isononyl isononanoate : | 3 à 5 g |
| DC 9510: Dimethicone/Vinyl Dimethicone Crosspolymer and C12-14 Pareth-12: | 1 à 2 g |
| Aqaxyl :Seppic :xylitylglucoside,anhydroxylitl,xylitol : | 0,8 à 1,2 g |
| Huile d'abricot raffinée : | 10 à 14 g |
| Sharomix 721 : dehydroacetic acid / benzyl alcohol : | 0,6 à 1 g |
| Simulgel EG: Seppic : sodium acrylate/sodium acryloyl methyltaurate, isohexadecane , polysorbate 80 : | 2 à 3 g |
| Sepinov EMT 10: Seppic : hydroxyethylacrylate/ sodium acryloyldimethyltaurate copolymer : | 0,8 à 1,2 g |
| Eau déminéralisée QSP | 100 g |

**-Exemple 4 : Pro acne gel blue**

| Principes actifs : | |
|---|---|
| Niacinamide : | 1 g |
| Acide oléïque : | 0,5 g |
| Dimethyl isosorbide : | 2 g |
| Calamine: | 0,3 g |

| Autres excipients : | |
|---|---|
| Osmocide 3 : Sederma : glycérine, eau, PEG 8, caprylylglycol sodium acrylate : | 28 à 32 g |
| Zinc glycinate : | 0,1 g |
| Glycérine: | 18 à 22 g |
| Sepinov EMT 10: Seppic :hydroxyethylacrylate/sodium acryloyldimethyltaurate copolymer : | 0,8 à 1,2 g |
| Syner GX : Soliance : cyamopsis tetragonoloba (gomme de guar) et gomme de xanthane: | 0,8 à 1,2 g |
| Sharomix 721: dehydroacetic acid /benzyl alcohol: | 0,8 à 1,2 g |
| Bleu covarine w6795 (CI 74160) : | 0,001 à 0,002 g |
| Eau déminéralisée QSP : | 100 g |

**- Exemple 5 : Pro acne gel red**

| Formulation identique à l'ex : 4, mais avec un colorant rouge | |
|---|---|
| Rouge covarine WN 3798 (CI 77491) : | 0,5 à 1 g |

-B : Les compositions topiques utilisées pour traiter les troubles de la pigmentation et plus particulièrement : éclaircissement du teint, taches brunes, lentigos solaires, éphélides et lucites selon la présente invention sont illustrées par les exemples 6 à 10 non limitatifs suivants :

**-Exemple 6 : Whitening corrector cleanser**

| Principes actifs : | |
|---|---|
| Niacinamide : | 0,35 g |
| Acide oléique : | 0,7 g |
| Dimethyl isosorbide : | 0,14 g |
| Acide octadécenedioïque : | 0,7 g |

| Autres excipients : | |
|---|---|
| Glycérine : | 0,4 à 0,6 g |
| Osmocide 3 : Sederma : glycérine, eau, PEG 8 caprylylglycol sodium polyacrylate : | 4 à 6 g |
| Simulgel: Seppic: sodium acrylate/sodium acryloylmethyl taurate, isohexadecane , polysorbate 80 : | 2 à 3 g |
| Sepinov EMT 10: Seppic : hydroxyethylacrylate / sodium acryloyldimethyltaurate coplymer : | 0,5 à 1,5 g |
| DUB ININ B : isononyl isononanoate | 2 à 4 g |
| Huile d'abricot raffinée : | 8 à 10 g |
| DehytonKcos BASF :cocamidopropylbetaine | 2 à 5 g |
| Proteol oat PF: Seppic : sodium lauroyl oat amino acids | 2 à 5 g |
| Texapon nso-is: sodium laureth sulfate | 3 à 5 g |
| Crothix liquid-Iq: Croda: PEG 150 pentaerythrithyl tetrastearate, PEG 6 caprylic/capricglycerides : | 0,4 à 0,6 g |
| Sharomix 721 : dehydroacetic acid/ benzylalcohol : | 0,6 à 0,8 g |
| Eau déminéralisée QSP : | 100 g |

**-Exemple 7: Whitening corrector cream**

| Principes actifs : | |
|---|---|
| Niacinamide : | 2 g |
| Acide oléique : | 2 g |
| Dimethyl isosorbide : | 1 g |
| Acide octadecenedioïque : | 2 g |
| Undecylenoyl phenylalanine : | 1 g |

| Autres excipients : | |
|---|---|
| Osmocide 3: Sederma : glycérine, eau, PEG 8, caprylylglycol, sodium polyacrylate : | 4 à 7 g |
| PW LS 9860 : BASF: maltodextrin,sucrose dilaurate, sodium cocoylglutamate, pisum sativum (pea/extract) | 0,5 à 1,5 g |
| DUB ININ B :isononyl isononanoate | 3 à 4 g |
| DC 9510 : Dimethicone/ Vinyl Dimethicone Crosspolymer and C12-14 Pareth-12 | 1 à 2 g |
| Aquaxyl: Seppic : xylitylglucoside, anhydroxylitol, xylitol : | 0,5 à 1,5g |
| Huile d'abricot raffinée : | 10 à 14 g |
| Polysorbate 60 : | 3,5 à 5,5 g |
| Sharomix 721 :dehydroacetic acid/ benzylalcohol : | 0,6 à 1 g |
| Sepinov EMT 10: Seppic :hhydroxyethylacrylate / sodium acryloyldimethyltaurate copolymer : | 0,5 à 1,5 g |
| Simulgel EG: Seppic : sodium acrylate/sodium acryloyl methyltaurate, isohexadecane , polysorbate 80 : | 1 à 3 g |
| Eau déminéralisée : QSP | 100 g |

**-Exemple 8: Whitening corrector serum**

| Principes actifs: | |
|---|---|
| Niacinamide: | 1 g |
| Acide oléique: | 2 g |
| Dimethyl isosorbide : | 1 g |
| Acide octadecenedioïque: | 2 g |
| Undecylenoyl phenylalanine : | 2 g |

| Autres excipients : | |
|---|---|
| Osmocide 3 : Sederma : glycérine, eau, PEG 8, caprylylglycol, sodium polyacrylate : | 4 à 5 g |
| PW LS 9860: BASF: maltodextrine, sucrose dilaurate, Sodium cocoylglutamate, pisum sativum (pea extract) | 1 à 3 g |
| DUB ININ B: isononyl isononanoate : | 3 à 5 g |
| DC 9510:Dimethicone /Vinyl Dimethicone Crosspolymer and Pareth -12 | 1 à 2 g |
| Aquaxyl : Seppic : xylitylglucoside, anhydroxylitol, xylitol : | 0,5 à 1,5 g |
| Huile d'abricot raffinée : | 11 à 13 g |
| Sharomix 721: dehydroacetic acid/benzylalcohol : | 0,6 à 1 g |
| Simulgel EG: Seppic : sodium acrylate/sodium acryloyl methyltaurate, isohexadecane , polysorbate 80 : | 0,4 à 0,8 g |
| Sepinov EMT 10: Seppic : hydroxyethylacrylate / sodium acryloyldimethyltaurate copolymer : | 0,2 à 0,6 g |
| Eau déminéralisée : QSP | 100 g |

**-Exemple 9: Pro Whitening corrector gel violet**

| Principes actifs : | |
|---|---|
| Niacinamide : | 1 g |
| Acide oléique : | 0,5 g |
| Dimethyl isosorbide : | 2 g |
| Acide octadécenedioïque : | 2 g |
| Undecylenoyl phenylalanine: | 2 g |

| Autres excipients : | |
|---|---|
| Osmocide 3 : Sederma : glycérine, eau, PEG 8, caprylylglycol, sodium polyacrylate : | 25 à 35 g |
| PW LS 9860 : BASF : | 1 à 3 g |
| Glycerine : | 15 à 25 g |
| Sepinov EMT 10 : Seppic : hydroxyethylacrylate/ sodium acryloyldimethyltaurate copolymer : | 0,6 à 1 g |
| Syner GX : soliance : cyamopsis tetragonoloba Gomme de guar/gomme de xanthane : | 0,6 à 1 g |
| Sharomix 721 : dehydro acetic acid/ benzylalcohol : | 0,8 à 1,2 g |
| Violet covarine W 5793 (CI : 51319) | 0,5 à 1 g |
| Eau déminéralisée : QSP | 100 g |

**-Exemple 10: Pro Whitening corrector gel rouge**

| Formulation identique à l'ex : 9, mais avec un colorant rouge | |
|---|---|
| Rouge covarine WN 3798 (CI 77 491) : | 0,5 à 1 g |

### Etude in vitro : Potentialisation de la Niacinamide par l'association de l'acide oléique et du diméthylisosorbide.

Conformément à la présente invention, il a été mis en évidence une potentialisation de l'activité anti-inflammatoire du niacinamide. Cette synergie a été objectivée par une évaluation de la baisse de production de prostaglandines 6KF1 alpha par le kératinocyte humain.

Sur ce modèle , on a évalué l'activité de la niacinamide en association avec l'acide oléique et le diméthylisosorbide sur la production de PG6KF1 alpha induite chez le kératinocyte par un stimulant de la cascade arachidonique, le ionophore calcique A23187 .

Le protocole est le suivant :
La suspension de kératinocytes humains HaCat dans le RPMI (milieu de culture cellulaire) 10 % SVF (sérum de veau foetal) est distribuée dans des plaques de 6 puits et incubée pendant 16 heures à 37 °C dans une atmosphère à 5 % de CO2. Les kératinocytes sont rincés avec du PBS (phosphate buffered salin) pour éliminer les cellules non adhérentes, puis exposés aux produits à tester inclus dans du RPMI sans SVF.

Les lots suivants ont été réalisés :
- Lot témoin
- Lot témoin stimulé par A23187 5 µM
- Acide oléique et diméthylisosorbide (1/0,25) 30 µg/ml + A23187 5 µM
- Niacinamide 20 µg/ml + A23187 5 µM

Les cellules sont incubées pendant 1 heure, avec les produits à tester, puis le ionophore calcique A 23187 est ajouté pendant 5 heures à la concentration de 5 µM.

Les milieux de culture de chacun des puits sont ensuite récupérés, centrifugés à 3000 tr/mn et conservés à - 80 °C.

La production de prostaglandine 6KF1 alpha pour chacun des essais est mesurée par Kit Elisa Euromedex.

Les résultats de cette étude sont exprimés dans le tableau suivant : en picogrammes de prostaglandines 6KF1 alpha pour 1x 10 puissance 6 kératinocytes / par ml de culture.

| Traitements | PG6KF1 alpha | % activité |
|---|---|---|
| Témoin | 35,23 | |
| Témoin + A23187 5 µM | 112,22 | |
| Acide oléique et dimethylisosorbide (1/0,25) 30 µg/ml + A23187 5 µM | 106,02 | - 5 % |
| Niacinamide 20 µg/ml + A23187 5 µM | 80,12 | - 28 % |
| Acide oléique et diméthylisosorbide (1/0,25) 30 µg/ml + Niacinamide 20 µg/ml + A23187 5 µM | 53,15 | -52 % |

Les résultats obtenus par cette étude in vitro montrent que la niacinamide présente une activité anti inflammatoire significative de (- 28 %).

La niacinamide avec l'association d'acide oléique et de diméthylisosorbide montre une activité synergique (- 52 %) d'inhibition par rapport (-33%) de l'effet additif, soit une nette potentialisation de l'action anti-inflammatoire.

### Etude clinique :

L'étude clinique a été réalisée sur 60 personnes de 18 à 35 ans souffrant d'acné, les trois formulations suivantes ont été testées :
- Formulation de l'exemple 1 Acné Cleanser (A)
- Formulation de l'exemple 1 sans l'acide oléique et diméthylisosorbide (B)
- Formulation de l'exemple 1 sans le Niacinamide (C)

Chaque formulation a été appliquée une fois par jour à la dose de 2 g /j sur 20 personnes pendant 2 mois.

Les critères primaires d'efficacité étaient :
- Le taux de succès, défini comme le pourcentage de patients considérés guéris, c'est-à-dire que le patient n'a plus de lésions acnéiques, ni comédons, ni de lésions inflammatoires, ou presque guéris.
- La réduction du pourcentage de lésions inflammatoires.

Les résultats sont présentés dans le tableau suivant :

| | Formulation A | Formulation B | Formulation C |
|---|---|---|---|
| Taux de succès | 24,2 % | 12,1 % | 6,4 % |
| Nombre de lésions inflammatoires | - 57,3 % | - 25,4 % | -15,1 % |

Ces résultats confirment la nette potentialisation de l'association Niacinamide avec l'acide oléique et le diméthylisosorbide comme anti-inflammatoire dans le traitement de l'acné.

De plus, un léger éclaircissement du teint a été observé chez les patients lors du traitement avec les formulations A et B. C'est la raison pour laquelle l'acide octadécenedioïque et/ou l'undecylenoyl phenylalanine sont ajoutés dans les formulations 6 à 10 utilisées pour le traitement des troubles de la pigmentation.

Remarque: pré- étude clinique réalisée sur 6 cas

Les patients sont toujours traités avec la crème de l'exemple 1, mais avec en plus des séances de LED thérapie aux longueurs d'ondes de 475 et 630 nm.

Cette méthode de traitement complémentaire permet d'obtenir un gain de 30 à 50 % en temps par rapport aux résultats obtenus avec l'utilisation de la crème seule.

## Revendications

1. Composition topique comprenant : de la niacinamide, de l'acide oléique et du diméthylisosorbide.

2. Composition selon la revendication 1, comprenant en outre de la calamine.

3. Composition selon la revendication 1 à 2, dans laquelle sont ajoutés un ou plusieurs agents dépigmentants.

4. Composition selon l'une quelconque des revendications 1 à 3, **caractérisée en ce que** la concentration en niacinamide est comprise entre 0,3 % et 3 % en poids.

5. Composition selon l'une quelconque des revendications 1 à 4, **caractérisée en ce que** la concentration en acide oléique est comprise entre 0,3 % et 3 % en poids.

6. Composition selon l'une quelconque des revendications 1 à 5, **caractérisée en ce que** la concentration en diméthyl isosorbide est comprise entre 0,1 % et 2 % en poids.

7. Composition selon l'une quelconque des revendications 2 à 6 **caractérisée en ce que** la concentration en calamine est comprise entre 0,3 % et 7 % en poids.

8. Composition selon l'une quelconque des revendications 3 à 7, **caractérisée en ce que** les agents dépigmentants sont l'acide octadécenedioïque et/ou l'undecylenoyl phenylalanine.

9. Composition selon la revendication 8, **caractérisée en ce que** la concentration en acide octadécenedioïque est comprise entre 0,5 % et 2 % en poids.

10. Composition selon les revendications 8 ou 9, **caractérisée en ce que** la concentration en undécylenoyl phénylalanine est comprise entre 1 % et 2 % en poids.

11. Composition selon l'une quelconque des revendications 1 à 10 pour son utilisation dans le traitement des troubles cutanées tels que l'acné ou les troubles de la pigmentation.

12. Procédé de traitement cosmétique, non thérapeutique, qui consiste à appliquer sur la peau d'un sujet une composition selon l'une des revendications 1 à 10 pour éclaircir la peau du sujet, et/ou éliminer les imperfections cutanées.

## Patentansprüche

1. Topische Zusammensetzung, aufweisend: Niacinamid, Oleinsäure und Dimethylisosorbid.

2. Zusammensetzung nach Anspruch 1, ferner aufweisend Galmei.

3. Zusammensetzung nach Anspruch 1 bis 2, in die eines oder mehrere Depigmentierungsmittel zugegeben werden.

4. Zusammensetzung nach einem der Ansprüche 1 bis 3, **dadurch gekennzeichnet, dass** die Niacinamidkonzentration zwischen 0,3 und 3 Gewichtsprozent beträgt.

5. Zusammensetzung nach einem der Ansprüche 1 bis 4, **dadurch gekennzeichnet, dass** die Oleinsäurekonzentration zwischen 0,3 und 3 Gew.-% beträgt.

6. Zusammensetzung nach einem der Ansprüche 1 bis 5, **dadurch gekennzeichnet, dass** die Dimethylisosorbidkonzentration zwischen 0,1 und 2 Gew.-% beträgt.

7. Zusammensetzung nach einem der Ansprüche 2 bis 6, **dadurch gekennzeichnet, dass** die Galmeikonzentration zwischen 0,3 und 7 Gew.-% beträgt.

8. Zusammensetzung nach einem der Ansprüche 3 bis 7, **dadurch gekennzeichnet, dass** die Depigmentierungsmittel Octadecansäure und/oder Undecylenoyl Phenylalanin sind.

9. Zusammensetzung nach Anspruch 8, **dadurch gekennzeichnet, dass** die Octadecansäurekonzentration zwischen 0,5 und 2 Gew.-% beträgt.

10. Zusammensetzung nach einem der Ansprüche 8 oder 9, **dadurch gekennzeichnet, dass** die Konzentration an Undecylenoyl Phenylalanin zwischen 1 und 2 Gew.-% beträgt.

11. Zusammensetzung nach einem der Ansprüche 1 bis 10 zur Verwendung bei der Behandlung von Hauterkrankungen wie etwa Akne oder Pigmenterkrankungen.

12. Verfahren zur kosmetischen, nicht-therapeutischen Behandlung, bestehend aus dem Auftragen einer Zusammensetzung nach einem der Ansprüche 1 bis 10 auf die Haut einer Person zum Aufhellen der Haut einer Person und/oder dem Beseitigen von Makeln.

## Claims

1. Topical composition comprising: niacinamide, oleic acid and dimethyl isosorbide.

2. Composition according to claim 1, further comprising calamine.

3. Composition according to claim 1 to 2, wherein one or several depigmentation agents are added.

4. Composition according to any of claims 1 to 3, **characterised in that** the concentration in niacinamide is between 0.3% and 3% by weight.

5. Composition according to any of claims 1 to 4, **characterised in that** the concentration in oleic acid is between 0.3% and 3% by weight.

6. Composition according to any of claims 1 to 5, **characterised in that** the concentration in dimethyl isosorbide is between 0.1% and 2% by weight.

7. Composition according to any of claims 2 to 6 **characterised in that** the concentration in calamine is between 0.3% and 7% by weight.

8. Composition according to any of claims 3 to 7, **characterised in that** the depigmentation agents are octadecenedioic acid and/or undecylenoyl phenylalanine.

9. Composition according to claim 8, **characterised in that** the concentration in octadecenedioic acid is between 0.5% and 2% by weight.

10. Composition according to claims 8 or 9, **characterised in that** the concentration in undecylenoyl phenylalanine is between 1% and 2% by weight.

11. Composition according to any of claims 1 to 10 for its use in the treatment of skin disorders such as acne or pigmentation disorders.

12. Method of cosmetic non-therapeutic treatment, which consists in applying on the skin of a subject a composition according to one of claims 1 to 10 in order to lighten the skin of the subject, and/or eliminate skin imperfections.
